# EUROPÄISCHE PATENTSCHRIFT

(11) **EP 1 181 563 B1**
(45) Veröffentlichungstag und Bekanntmachung des Hinweises auf die Patenterteilung: **26.07.2006**
(21) Anmeldenummer: 99955628.5
(22) Anmeldetag: 18.11.1999
(51) Int. Cl.: G01R 29/08

(54) **Verfahren und Messeinrichtung zur Erfassung von elektromagnetischen Störfeldern**
Method and measuring device for determining electromagnetic interference fields
Procédé et dispositif de mesure pour déterminer des interférences électromagnétiques

(30) Priorität: 18.11.1998 CH 230798; 14.07.1999 CH 129899
(43) Veröffentlichungstag der Anmeldung: 27.02.2002
(73) Patentinhaber: Ackermann Patent GmbH, 9231 Egg-Flawil (CH)
(72) Erfinder: HENGSTENBERG, Werner, D-88260 Argenbühl-Siggen (DE); ACKERMANN, René, CH-9231 Egg-Flawil (CH)
(74) Vertreter: Ackermann, Ernst
(86) Internationale Anmeldenummer: PCT/CH1999/000551
(87) Internationale Veröffentlichungsnummer: WO 2000/029859

(56) Entgegenhaltungen:
- WO-A-96/35371
- DE-A- 19 636 277
- DE-U- 29 504 522
- NEUMANN M: "ELEKTROSMOG MESSEN" FUNKSCHAU,DE,FRANZIS-VERLAG K.G. MUNCHEN, Nr. 11, 9. Mai 1997 (1997-05-09), Seiten 78-81, XP000728950 ISSN: 0016-2841

## Beschreibung

### Technisches Gebiet

Die Erfindung betrifft ein Verfahren zur Erfassung von hochfrequenten elektromagnetischen Störwechseifeldem über eine Antennenspannung mittels batteriegespiesenen Messgeräten. Die Erfindung betrifft ferner eine Handmesseinrichtung mit Batteriespeisung für die Erfassung von hochfrequenten elektromagnetischen Störwechseffeldem über eine Antennenspannung mit einer Digitalanzeige und/oder Bildanzeige und mit einem Antennenanschluss. Die Erfindung betrifft des weiteren die Verwendung der Handmesseinrichtung für die Messung und Überwachung der Immission in Bezug auf die Zeit sowie die drahtlose Sendetechnik.

### Stand der Technik

Die neue Lösung betrifft den Bereich der sogenannt nicht- oder a-thermischen Wirkung von elektromagnetischen Feldern. Hier macht die Erfassung eines Leistungsgleichwertes z.B. in W/cm² wenig Sinn. Erfindungsgemäss ist erkannt worden, dass jeder Messwert in direkter oder indirekter Beziehung zu spezifischen Störquellen und ihren Störwirkungen sein soll. Verschiedenste sendetechnisch spezifische Wirkungen sind bekannt. Dies macht eine Vielzahl von Messgeräten erforderlich. Daraus resultieren ein enormer finanzieller Aufwand und grosse Schwierigkeiten mit jedem Gerät entsprechend im Nieder-, Mittel- und Höchstfrequenzbereich umzugehen, und das Ergebnis im Sinne einer Diagnose verwerten zu können. Der Aufwand bis sich ein Spezialist in die Lage versetzt hat, enormen Messungen machen zu können, ist enorm. Es müssen zudem hohe Geldbeträge verlangt werden, damit die entsprechenden Messaufwendungen abgedeckt sind. Es gibt im Zeitpunkt der neuen Anmeldung nur einen kleinen Personenkreis, der in der Lage ist, eine Belastungssituation im Hinblick auf den ganzen Bereich Elektrosmog, wie heute dieser Belastungskomplex ganz verschwommen bezeichnet wird, auszumessen.

In der sogenannten elektrobiologischen Messpraxis werden die verschiedensten Verfahren sowie Messgerätschaften eingesetzt, ohne dass sich ein allgemeines Konzept für den ganzen Bereich Elektrosmog, um mit kostengünstigen Handmessgeräten ausmessen zu können, etabliert hat. Es werden je unterschiedliche Messgeräte für den Bereich der Niederfrequenz sowie die Hochfrequenz angeboten. Die DE-OS 196 36 277 schlägt eine Vorrichtung zur Detektion elektromagnetischer Wechselfelder in einem zwischen 1 Hz und 20 kHz liegenden Frequenzband vor. Dabei wird eine Antenne zum Empfang der elektromagnetischen Wechselfelder oder einen Anschluss für eine solche Antenne, wobei wenigstens eine Verstärkungseinrichtung die von der Antenne breitbandig empfangenen Signale in gewünschtem Frequenzband verstärkt. Ferner ist eine Einrichtung zum Umwandeln der verstärkten elektrischen Signale in akustische Signale gleicher Frequenz vorgesehen.

Das DE-GM 295 04 522 bezieht sich auf ein batteriebetriebenes Handgerät zum Auffinden von elektrischen und/oder magnetischen Nahfeldern ebenfalls im niederfrequenten Bereich. Eine elektronische Schaltungsanordnung zur separaten Erfassung eines elektrischen und eines magnetischen Feldes, deren jeweilige Suchorgane in Suchrichtung des Gerätes angebracht sind, sind definiert zueinander angeordnet und für eine ausgangsseitige Signalgabe einzeln oder gemeinsam aktivierbar.

Die WO96/35371 umfasst neben der klassischen Niederfrequenz zusätzlich einen mittleren Frequenzbereich und misst die Körperspannung. Es wird eine Messhilfe vorgeschlagen, für eine unverfälschte Messung für den Bereich von niederen Aufladungen am menschlichen Körper, vor allem an der Haut des Menschen. Die Messhilfe ist an eine Erde anschliessbar und als Wechselspannungsmessgerät ausgebildet und weist eine festangebrachte bzw. anbringbare Hautkontaktstelle auf, zur Messung und Anzeige der unverfälschten elektrischen Wechselspannung direkt am menschlichen Körper.

In der Praxis werden für den Hochfrequenzbereich, z.B. von 100 kHz bis 3 GHz, sogenannte professionelle Geräte eingesetzt. Es handelt sich um Geräte, welche für die elektromagnetische Verträglichkeit (EMV) entwickelt wurden. Es wird dabei von staatlich vorgegebenen Grenzwerten ausgegangen, welche in Bezug auf die Anforderungen der elektrobiologsichen Messpraxis im Faktor 100 - 1000 zu hoch festgelegt sind. Mit den meisten bekannten EMV Geräten kann der Mikrovoltbereich für die Antennenspannung nicht mehr gemessen werden.

Für den Bereich Hochfrequenz wird Bezug genommen auf den Fachartikel Neumann: "Elektrosmog Messen" Funkschau DE Franzis-Verlag K.G, München Nr. 11, 9. Mai 1997, Seiten 78 - 81: betreffend beiden Messgeräte Wandel & Goltermann und Chauvin Amoux. Bei hochfrequenten Feldern finden meist Antennen aus kleinen Flächendipolen Verwendung, die durch eine ausgeklügelte Anordnung von Widerständen stark bedämpft werden. So entsteht eine konstante Ausgangsspannung von 100 kHz bis 3 GHz, die aber auf Kosten der Empfindlichkeit erreicht wird. Verglichen mit normalen Antennen beträgt die Ausgangsspannung nur einen Bruchteil und deshalb funktionieren diese Anordnungen nur bis herab zu etwa 1 Volt/Meter. Eine Schottky-Diode direkt am Dipol richtet die Spannung gleich, die dann über eine Widerstandsleitung an den Eingang der Anzeige-Elektronik weitergereicht wird, um Verzerrungen des Feldes zu vermeiden.

### Darstellung der Erfindung

Der Erfindung wurde nun die Aufgabe gestellt, nicht nur nach einer vereinfachten Messtechnik zu suchen, sondern wenn möglich auch Belastungskriterien zu definieren, die erlauben über den ganzen Bereich Elektrosmog eine belastungsreelle Aussage in Bezug auf Lebewesen vor allem auch für den Menschen machen zu können, und gegebenenfalls notwendige Sanierungsmassnahmen rasch ergreifen und kontrollieren zu können. Es war ferner ein wichtiger Teil der Erfindung, unter Vermeidung des Einsatzes eines "Messlabors" bzw. eines teuren Messwagens, wie etwa bei der Überprüfung von Sendestörproblemen, eine effiziente Hausmesstechnik mit reproduzierbaren Messresultaten zu entwickeln.

Das erfindungsgemässe Verfahren zur Erfassung von hochfrequenten elektromagnetischen Störwechselfeldern über eine Antennenspannung mittels eines batteriegespeisten Messgerätes mit einer Eingangsempfindlichkeit bis in den Mikrovoltbereich, mit einer Digital- und /oder Bildanzeige und mit einem Antennenanschluss ist mit folgenden Schritten gekennzeichnet:
a) Erfassung eines hochfrequenten Träger-Störwechselfeldes
b) Erfassung von Peakwerten des Störwechselfeldes
c) Erfassung einer niederfrequenten Modulation des Störwechselfeldes im Bereich von 0 bis etwa 15 kHz.

Die erfindungsgemässe Handmesseinrichtung ist dadurch gekennzeichnet, dass die Messeinrichtung eine Eingangsempfindlichkeit bis in den Mikrovoltbereich aufweist und geeignet ist für die Erfassung der Peakwerte des Störwechselfeldes, wobei mit der Messeinrichtung sowohl
a) ein hochfrequentes Träger-Störwechselfeld als auch
b) eine niederfrequente Modulation des Störwechselfeldes im Bereich von 0 bis 15 kHz erfassbar ist.

Das Handmesssystem besteht aus einer Handmesseinrichtung und einer Schleifenantenne, ist dadurch gekennzeichnet, dass die Schleifenantenne als abgeschirmtes Leiterstück ausgebildet ist, mit einer Abschirmung in Bezug auf ein elektrisches Feld, wobei die Schleifenantenne etwa in Handgrösse ausgebildet ist, und eine runde oder elliptische Form hat, und/oder das zwei oder mehrere Schleifenantennen mit einer wirksamen Antennendrahtlänge in dem Bereich von 10 bis 60 cm einsetzbar sind.

Ferner wird die Verwendung der Handmesseinrichtung für die Messung und Überwachung der Immission in Bezug auf die Zeit sowie die drahtlosen Sendetechnik wie Mobilfunk, drahtlosen Haustelefon, TV-Sendestrahlung und den übrigen Sendesystemen im Megahertzbereich bis zu einem Bereich 10 Gigahertz, insbesondere mit akustischer und/oder bildhafter Anzeige der Modulation und im besonderen der Peak-Werte vorgeschlagen.

Mit der Spannungsmessung in Volt bzw. Milli- oder Mikrovolt ergibt sich gleichsam ein Bindeglied über den gesamten Frequenzbereich. Die erhaltenen Werte haben in Verbindung mit wenigstens einem oder zwei weiteren Parametem eine extrem hohe Aussagekraft für eine Belastungssituation auf Lebewesen. Die Spannung in Volt ist ein Wert, der aus dem Alltag jedermann bekannt ist, sei es von der Netzspannung, der Spannung einer Taschenlampenbatterie oder als biologische Potentialspannung die in jedem medizinischen Nachschlagwerk (in Millivolt-Mikrovolt) dargestellt sind. Jeder Berufsmann hat einen Bezug zur elektrischen Spannung und den verschiedenen Spannungsbereichen bzw. kann diese leicht herstellen. Erfindungsgemäss wird jedoch ein weiteres Kriterium nämlich "leiterbegleitende" miteinbezogen. Es ist eine Tatsache, dass elektromagnetische Schwingungen bis zu einer bestimmten Frequenz über Leiter über grosse Distanzen übertragen werden können (bis ca. 1 - 3 MHz). Dagegen lassen sich höhere Frequenzen über 1 MHz nicht mehr einfach über normale Kupferleitungen übertragen. Der viel wichtigere Grund weshalb leiterbegleitend mit einbezogen wird, liegt darin, dass die Abschirmtechnik in den beiden Bereichen völlig anders ist. Niedere Frequenzen werden nicht nur über Leiter, sondern auch über viele Baumaterialien übertragen, bzw. werden durch diese mehr oder weniger gedämpft bzw. abgeschirmt. Hohe und höchste Frequenzen mehrheitlich von Sendeanlagen ausgestrahlt, dringen durch die Fenster eines Hauses in die Wohn- und Arbeitsräume. Hier kann nur mit spezifischen Massnahmen entgegengewirkt werden. Die Messtechnik ist ganz vordergründig auch auf die jeweils mögliche Sanierung einer Situation ausgerichtet. Es entsteht ein Messbewusstsein, indem die Messwerte sofort besondere Lösungen beinahe suggerieren. Damit ist es gelungen für den ganzen Frequenzbereich den Pegel der Einwirkung der Störbelastung zu erfassen, wobei der Aussagewert sehr hoch ist. In der konkreten Ausgestaltung bedeutet dies, dass im niederfrequenten Bereich, sei es in zwei Geräten oder je in einem einzigen Gerät die zwei zentralsten Wirkungen erfasst werden. Im niederfrequenten Bereich ist es vor allem das elektrische und das magnetische Feld und im höchstfrequenten Bereich die örtliche Antennenspannung z.B. im Mikrowellenbereich und als Ergänzung kann z.B. eine akustische Umsetzung für das Erkennen der spezifischen Signaltechnik (digital, gepulst, getaktet usw.) erfasst werden. Die erste Frage nach dem wieviel wird über eine Spannungsangabe und gegebenenfalls daraus errechneten Feldwerten und die zweite Frage nach der Wirkintensität in der Anzeige des örtlichen Magnetfeldes bzw. der örtlich wirksamen Störsignaltechnik festgestellt. Gerätetechnisch sind alle genannten Faktoren, mittels preisgünstigen, einfachen, gegebenenfalls sogar in kombinierten Handmessgeräten zu handhaben.

Durch den unterschiedlichen Messaufbau für niedere und hohe Frequenzen ergeben sich vorrichtungsgemäss zwei Messaufnehmer. Zuerst der niederfrequente Teil. Im Gegensatz zu dem elektrischen Schutzleiter, bei dem primär der Widerstand (in Ohm) geprüft wird, kann gemäss der neuen Lösung bei der biologischen Erdung die sehr viel problemlosere Messung der Spannung in Volt bzw. Millivolt als Prüfmethode gewählt werden. In den weit überwiegenden Fällen kann die Oberflächenerdung über einen Erdungspfahl als Basis und Vergleichswert für die anderen Potentiale genommen, die optimalen Erdungsverhältnisse gesucht und für ein biologisch günstiges Arbeits- oder Schlafumfeld benutzt werden. Die Erfindung erlaubt eine sehr wirksame Strategie für die Eliminierung von hausgemachtem NF-Elektrosmog und aber auch in Bezug auf verseuchte Schutzleiter. Die biologische Erdungsmöglichkeiten wird über ein hochohmiges Voltmeter geprüft entsprechend ausgewählt. Für das hochohmige Voltmeter wird auf die WO96/35371 Bezug genommen.

Erfindungsgemäss sind für den NF-Bereich ganz besonders sechs Problemkreise erkannt worden: **A. Potentialausgleich:** Der Potentialausgleich schliesst Störfelder im mittleren Frequenzbereich mit ein. Der Potentialausgleich ist deshalb sehr wichtig, weil alle nichtgeerdeten und elektrisch leitenden Teile in einem Haus bzw. Raum sich an das entsprechende Störfeld ankoppeln können und selbst wieder eine Störquelle bilden. **B. Biologische Erdung**: Jede Messung setzt klare Bedingungen und im Falle einer Spannungsmessung ein definiertes Ausgangspotential voraus. Häufig wurde bei Elektrosmogmessungen von dem Potential des Schutzleiters der Elektroinstallation ausgegangen, dies obwohl bekannt ist, dass Schutzleiter vielfach Wechselspannungen aufweisen. Es darf nicht vorausgesetzt werden, dass der elektrische Schutzleiter Potential Null hat. **C. Eingangswiderstand des Messgerätes:** Laborvoltmeter haben entsprechend internationalen Normen zwischen 1 und 25 Mega Ω Eingangswiderstand. Man geht davon aus, dass die Kapazität einer Person gegenüber Erde in typischen Wohnsituationen kleiner 100 pF ist. Dies entspricht einer Impedanz von 10 bis 100 Megaohm. Es hat sich nun gezeigt, dass sowohl in Bezug auf den Potentialausgleich wie auch die ganze Erdproblematik ähnliche Verhältnisse gelten. In beiden Fällen muss der Eingangswiderstand grösser als 100 Megaohm sein. **D. Ankopplungsspannung:** Überraschenderweise hat es sich gezeigt, dass sowohl die Messtechnik für die Ankopplungsspannung übertragbar ist auf das Gebiet der Messung unterschiedlicher Potentiale wie auch der biologischen Erdung. Dies bedeutet, dass gemäss der neuen Lösung mit ein und dem selben Gerät über entsprechende Anschlüsse alle drei Problemkreise mit hoher Genauigkeit messbar sind. **E. Oberschwingungen:** Als Oberschwingungen werden die auf einer Schwingfrequenz über- oder gegebenenfalls unterlagerten höher- oder tieferfrequenten Störschwingungen verstanden, dies zumindest bei der Stromversorgung, gewünscht wäre eine schöne sinusförmige Schwingung. Abgesehen von den verschiedensten Quellen für entsprechende Störungen besteht das Bestreben über die Stromversorgung digitalisiert und gepulst Informationssignale zu übertragen. Damit kommen zu den klassischen gleichsam unerwünschten Störsignalen auf der Niederfrequenzseite zusätzlich, zumindest im Sinne der elektrobiologie unerwünschte, reine Informationsstörsignale dazu, die mit klassischen Spannungsmessaufnehmern nicht mehr erfassbar sind.

Hochfrequenzseitig ist vom Erfinder entdeckt worden, dass HF-"Hot-spots" nicht nur wie bisher bekannt im Gehirn eines Menschen, sondern auch in Form von Störballungen zumindest innerhalb von Gebäuden häufig durch dominante Magnetfelder auftreten. Bereits bei ersten Messserien wurde mit einer Magnetfeldantenne sowie eine für die höchste Empfindlichkeit gebaute Elektronik eine überraschende Häufigkeit der angesprochenen Magnetfeldanomalien bzw. Phänomene festgestellt. Entsprechende Störquellen stammen meistens von Sendestrahlen. Unter Störballungen werden Konzentrationen von elektromagnetischer Strahlung, überwiegend im HF-Bereich verstanden. Je nach Frequenzgemisch und örtlichen Verhältnissen, können diese nadelkopfgrosse "Hot-spots" sein, und werden bei Mikrowellen, oder MobilfunkSendestrahlen immer häufiger auch als Frequenzgemisch festgestellt. In bewohnten Räumen stellt man zunehmend auch grössere Störballungen von Fussballgrösse, bis zu Metergrösse fest. Die magnetischen Komponenten dieser "HF-Störballungen" dringen vollständig in den menschlichen Körper ein und stellen im Falle von gepulsten Strahlen eine besondere Gefahr dar, insbesondere bei Langzeiteinwirkung. Die jüngste Erfahrung zeigt, dass ein Gleiches bei Tieren und Pflanzen, ja sogar die ganze Biologie in der Erde, im Wasser oder in der Luft gilt. Die Pulsstrahlung stört die Vorgänge in den Zellen. Der Bereich der Immissionen muss damit bei gepulsten Techniken wie dem Mobilfunk neu bewertet werten. Es ergeben sich grösserräumig gesehen, auch in einem Wohnquartier besonders durch beliebige Reflexionen vollständig chaotische Bedingungen, die zudem zeitlich starken Schwankungen unterworfen sind. Selbst in freier Natur stellt man nicht nur Grosskeulen von Grosssendern von gegebenenfalls mehreren hundert Metern Mächtigkeit, sondern auch feine und feinste Keulen, und wie weiter oben beschreiben, örtliche Ballungen und "Hot-spots" fest. Bereits in den Anfängen der Mikrowellenforschung wurde erkannt, dass sich Mikrowellen in Ausbrüchen, in Ballungen ausbreitet. Überall werden solche Ballungen oder Feldkonzentrationen festgestellt, dies in totalem Widerspruch der Theorie einer quadratischen Abnahme der Leistungsflussdichte. Sehr interessant ist ferner die Beobachtung von zunehmenden Feldstärken eine Art Feldstärkenstau, wenn eine Hanglage weit ab vom Sender direkt bestrahlt wird. Auch hier können wiederum örtliche Überhöhungen bzw. extreme Störballungen gemessen werden, z.B. in dm oder Metergrösse im Extremfall sogar mehreren 100 Metern. Ein weiteres Thema ist die Polarisation, welche als Vertikal und als Horizontalpolarisation erzeugt und messtechnisch durch senkrecht oder horizontal Halten einer Resonanz-antenne messbar ist. Ein absolut unerforschtes Gebiet ist nicht so sehr die theoretische Existenz sondern die mögliche Schadwirkung einer Kreuz- oder "Quasikreuzpolari-sation", insbesondere von unterschiedlichen jedoch nahe beieinander liegenden Frequenzen. Von den Erfindern ist erkannt worden, dass für das Verständnis der örtlichen Einwirkung von Elektrosmog besonders im HF-Bereich die aufgezeigte Realität, sei es von Sendehauptkeulen oder vielen 1000 Sendenebenkeulen und Kreuzungspunkteffekte gleicher und verschiedener Sendefrequenzen berücksichtigt werden müssen. Vor allem aber auch Verdrängungseffekte der verschiedenen Felder, Seitenbandeffekte z.B. 1, 2 und 3 Fresnelzonen. Fünf ganz besondere Gesichtspunkte haben sich auf Grund der neuen Lösung in dem Bereich der Hochfrequenz herauskristallisiert:
- Der Messgeräteaufbau mit dem Verstärkersystem muss eine sehr hohe Empfindlichkeit haben und wenigstens im Mikrovoltbereich noch Belastungswerte digital anzeigen. • Es sollen sowohl die elektrischen wie die magnetischen Feldanteile gegebenenfalls gesondert anzeigbar sein. • Entscheidend ist, dass unabhängig davon, ob die elektrische oder die magnetische Komponente gemessen wird, nicht nur der Träger mit Art- und Frequenz feststellbar ist, sondern gleichzeitig festgestellt wird, insbesondere um welche Modulationstechnik oder welche Taktfrequenzen in dem Bereich der Elf-Frequenzen örtlich einwirken (z.B. auch ob es sich um AM- oder FM-Technik handelt). • Es soll sofort die örtlich grösstmögliche Grösse des Störeffektes z.B. als Pfeifton (Mobilfunk) oder Rattern (z.B. TV-Bildsteuersignal) oder insbesondere auch die Intensität der Elf-Signale durch grössere oder geringere Lautstärke angezeigt werden. Dies ist besonders wichtig, in den Anwendungen z.B. vielen Haustechniken, bei denen das Trägerfeld nur noch minimale Werte im Mikrovoltbereich ergibt. Alle wichtigen Störquellen im anvisierten Frequenzbereich werden durch eine angepasste Breitbandigkeit und gegebenenfalls angepasste Antennenlängen mit hoher Genauigkeit erfasst. Die Information bzw. Störinformation für jegliches biologisches System soll in der Art und Intensität ermittelt werden.

Die neue Lösung eröffnet damit für die schnelle Erfassung der Störparameter für die Biologie eine überraschend einfache Hausmesstechnik. Weil nun die Messantenne gleichsam an die reale Feldform im Raume, als Kugel (in der idealisierten Annahme) angepasst ist, werden durch die Schleifenform sehr viel rascher als bisher örtliche Störballungen detektiert. Das Ziel jeder Hausmessung ist ja das Auffinden der grössten und kleinsten Belastungsstellen auf Lebewesen. Es ist also nicht irgend ein Mittel- oder Zufallswert, der gesucht wird sondern primär der Faktor des Störsignals bzw. der Störinformation. Die elektromagnetischen Ballungen erzeugen ohne Zweifel mit ihren Störinformationen die grösstmögliche Schadwirkung, besonders wenn sich z.B. ein Mensch, sei es mit der Herz- oder Kopfpartie exakt inmitten einer Störballung langzeitig aufhält. Der Mensch ist bestrebt, in jedem Raum sich tendenziell nahe der Fensterfront zu plazieren, genau dort, wo am häufigsten Störballungen feststellt werden. Die neue Erfindung erlaubt die Grösse der örtlichen Feldwirkung wie der darin enthaltenen Informationswirkung unabhängig und gleichzeitig, das heisst die örtlich jeweils grösste Störwirkung zu erfassen. Gemäss dem neuen Messverfahren werden in einem ersten Schritt vor allem inhomogene Feldwirkungen besonders auch solche mit magnetischer Feldwirkung mittels einer breitbandigen Ring- bzw. Schleifenantenne ermittelt. Dafür wird mit der Schleifenantenne der Raum in alle Richtungen abgeschritten und abgesucht. Orte hoher Feldstärken können nun auf mehrfache Weise sofort festgestellt:
- Erstens durch grössere Spannungswerte an der Geräteanzeige. Dabei kann die Feldstärke in V/m durch Multiplizieren leicht errechnet werden.
- Zweitens durch die Stärke und Art des Geräuschpegels, wenn die Elf-Modulationen akustisch angezeigt werden.
- Oder für Analysezwecke durch bildliches Aufzeigen der ELF-Modulationen.
Durch Drehen und Wenden des Messgerätes mit der Schleifenantenne beim Absuchen im Raum wird rasch die Mächtigkeit der Störballungen ermittelt. Darauf können im zweiten Schritt im hochfrequenten Bereich, insbesondere bei den cm, dm- und m-Wellen die Antennenspannung mittels verschiedenen Resonanzantennen mit grösserer Präzision festgestellt und in Bezug auf die niederfrequenten Störsignale die Art der Signaltechnik akustisch und gegebenenfalls bildlich ermittelt und die frequenzmässige Selektion z.B. durch den wirksamen Längenbereich der Resonanzantenne bestimmt werden. Bevorzugt wird die Praxismessung bzw. Hausmessung mit einem breitbandig arbeitenden Antennen-Spannungsmessgerät, durch Aufstecken verschieden grosser Antennen durchgeführt. Es eröffnet sich damit eine völlig neue Messpraxis. Bevorzugt wird auch die Schleifendetektorantenne extrem breitbandig konzipiert, so dass Frequenzen nahezu im ganzen MHz-Bereich (ab 10 bis 20 MHz) bis über 4 GHz sofort und zeitgleich erfasst werden können. Die fünf Schritte der neuen Messmethode sind stichwortartig: Detektieren, Analysieren, Diagnostizieren, Sanieren, Kontrollieren.

Der neue Lösungsweg verzichtet in einer ersten Phase im HF-Bereich ganz bewusst auf eine wissenschaftliche Analyse etwa in der Form von Spektrumsanalyser besonders mit Stativaufstellung. Im Vordergrund ist die Tondarstellung über einen Lautsprecher, mit dem die spezifische Modulationstechnik mehrheitlich sofort mit dem Gehör diagnostiziert wird oder mit einem Handoszilloskop mit Bilddarstellung. Die Erfindung zielt für das Aufsuchen der Belastungszonen zuerst in die Breite (breitbandig), das heisst das ganze hochfrequente Störspektrum gleichzeitig, mit bevorzugt akustischer Anzeige des oder der grossen Störstrahlenbelastungen bzw. Störmodulationen. Weil die Schleife optimal an die Störballungsform genau so wie an die einzelnen Körperteile (Kopf, Sonnengeflecht usw.) angepasst ist, ergeben sich bei Erfassung der magnetischen Komponente (mit Modulation) sofort die kräftigsten Störkomponenten. Von den Anmeldern werden alle technischen elektromagnetischen Strahlen oder Wellenwirkungen, bzw. Feldwirkungen als Störfelder bzw. Störfaktoren bezeichnet, insoweit diese den Zellhaushalt oder ganze biologische Kreisläufe in irgend einer Art negativ beeinflussen können. Bekannteste EMV-Geräte haben eine tiefste Empfindlichkeit von z.B. 0,1 V/m, im Extremfalle von 0,01 V/m. Dies entspricht einer Antennenspannung von etwa 2 mVolt bis 20 mVolt. Diese Werte liegen gerade über dem Bereich, in dem die grösste Häufigkeit der entdeckten Störballungen liegen. Die jüngste Erfahrung zeigt, dass elektrosensible Personen bereits bei Störfeldern ab 0,1 mV Antennenspannung reagieren, wenn es sich um gepulste Strahlung handelt. Die Modulation oder Pulstechnik wirkt dabei als Störinformation für biologische Systeme, besonders für die Zellfunktion so dass die Folge bis zu schwersten Krankheiten sein kann. Daraus leitet sich die Forderung einer entsprechenden Empfindlichkeit der Messgeräte ab.

Sehr wichtig ist, dass es sich bei der Schleifenantenne um eine nicht-abgestimmte Antenne handelt, die breitbandig etwa von 10 MHz bis 10 GHz das ganze Spektrum erfasst. Die Schleifenantenne liefert für die erste Messung einen Summenwert. Der Summenwert ist stark abhängig von der Grösse der Schleifenantenne und der wirksamen Schleifenlänge. Optimal ist etwa Hand- bis Kopfgrösse, das heisst eine gestreckte Länge in dem Bereich von z.B. 20 bis 60 cm. Die Antennenanschlüsse des Messgerätes werden mit Schnellwechselverbindungen ausgebildet, derart, dass wahlweise eine Schleifenantenne oder eine Resonanzantenne anschliessbar ist. Das Messgerät weist zu diesem Zweck bevorzugt zwei oder mehr, z.B. drei Antennenanschlüsse auf, für verschiedene Messbereiche, mit entsprechenden Bereichsschaltern. Die beiden Enden der Schleifenantenne sind zur Stabilisierung der Schleifenform an zwei versetzt angeordneten Antennenanschlüssen verbindbar, wobei einerseits der Massekontakt mit der Seele, und anderseits der Kontakt mit dem Signaleingang in die Messelektronik herstellbar ist. In dem Bereich des Antennenanschlusses zwischen Massekontakt und Seele der Schleifenantenne kann ein Schalter zur Unterbrechung der Verbindung angeordnet werden. Die Schleifenantenne ist als abgeschirmtes Leiterstück ausgebildet ist, mit einer genügend guten Netzabschirmung in Bezug auf das elektrische Feld. Gemäss einer weiteren Ausgestaltung der neuen Lösung ist zur Richtungsbestimmung örtlich einwirkender HF-Störstrahlen einer passenden Resonanzantenne ein V-förmiger Reflektortrichter anschliessbar, wobei der Reflektortrichter im Einsatz die Resonanzantenne über der ganzen Antennenlänge umfasst. Dies gestattet bei eindeutiger Strahlenrichtung die exakte Einstrahlzone rasch zu bestimmten, und den Effekt von Abschirmmassnahmen bei Hochfrequenz ebenso schnell zu kontrollieren.

Immer mehr werden bei der Hausmessung Frequenzanalysen oder zumindest eine dokumentarisches Erfassen der relevanten Störinformationen verlangt. Zur Weiterausgestaltung der neuen Lösung können als besonders bevorzugt die bisher nur im niederfrequenten oder nur im hochfrequenten Bereich darstellbaren Störfrequenzen über eine einzige Messeinrichtung ausgewertet werden. Der enorme Vorteil dabei ist, dass nun erstmals die im Milli- und Nanosekunden auftretenden Pulssignale bzw. Informationspakete auf dem Netzstrom genau so wie die ELF-Modulationen auf der Hoch- und Höchstfrequenz auffindbar und über die gleiche Hardware darstellbar sind. Ein entsprechend konzipiertes Oszilloskop wird mit den dafür notwendigen Anschlüssen für die Messaufnehmer ausgerüstet. Diese können die zuvor beschriebenen Messgeräte selbst oder speziell adaptierte Messaufnehmer sein, die sich auf eine sinngemässe Aufnahmetechnik stützen.

### Kurze Beschreibung der Erfindung

Die Erfindung wird nun an Hand von einigen Ausführungsbeispielen mit weiteren Einzelheiten erläutert. Es zeigen:
- die Figur 1a: beispielhaft einen Richtwertverlauf über dem zur Zeit wichtigsten Frequenzspektrum von Elektrosmog und den verfahrenstechnischen Einsatz der Messgeräte;
- die Figur 1b: eine Übersicht über den elektromagnetischen Bereich und den Hörbereich;
- die Figur 1c: bildhaft mögliche Resonanzfrequenzen beim Menschen;
- die Figur 1d: typische Schwingungsformen von NF-Quellen;
- die Figur 1e: typische Signalformen von HF-Quellen;
- die Figur 2: schematisch eine Sendekeule mit Seitenbänder;
- die Figur 3: Beispiel einer Schleifenantenne;
- die Figur 4a: bis 4d verschiedene Antennen zum wahlweisen Anschliessen an ein Messgerät;
- die Figur 5: Messgerät HF ohne Antenne mit drei Antennenanschlüssen;
- die Figur 6a bis 6d: verschiedene Dispositionen, Schleifenantenne (Figur 6b), aufgebaut auf ein Messgerät Figur 6a sowie eine Messsituation Figur 6c und die Figur 6d das Messgerät mit aufgesteckter Resonanzantenne;
- die Figur 7a: und 7b ein Antennenpaar für das Anpeilen der Strahlrichtung sowie schematisch eine Anpeildisposition;
- die Figur 8: ein Oszilloskop für die Darstellung und Auswertung der ELF-Frequenzen;
- die Figur 9a: bildlich die Zielvorstellung sowohl für die Elektrosmogmessung wie die Sanierung für den NF-Bereich;
- die Figur 9b: die Messung der Ankopplungsspannung über den Menschen mit Messung in Bezug auf Schutzleiter der elektrischen Installation;
- die Figur 9c: die rein gerätetechnische Messung der elektrischen Potentiale im Wohnraum, Beispiel: Potentialvergleich Schutzleiter, Wasserleitung, Radiator;
- die Figur 10a und 10b: ein neues NF-Messgerät, das in Kombination sowohl rein gerätetechnisch wie über den Menschen die Messung von Potentialunterschieden erlaubt.

### Wege und Ausführung der Erfindung

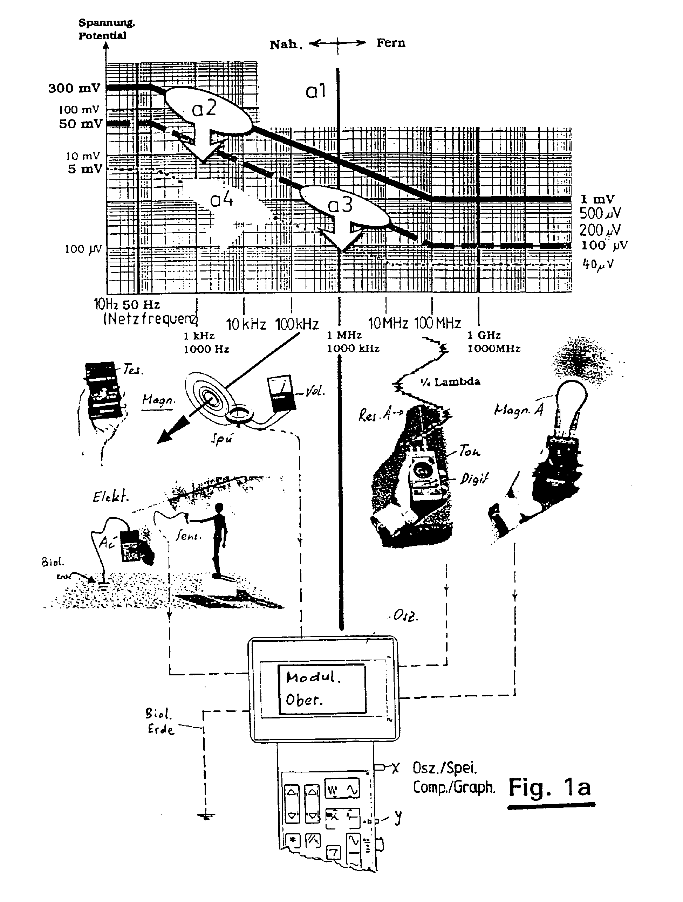

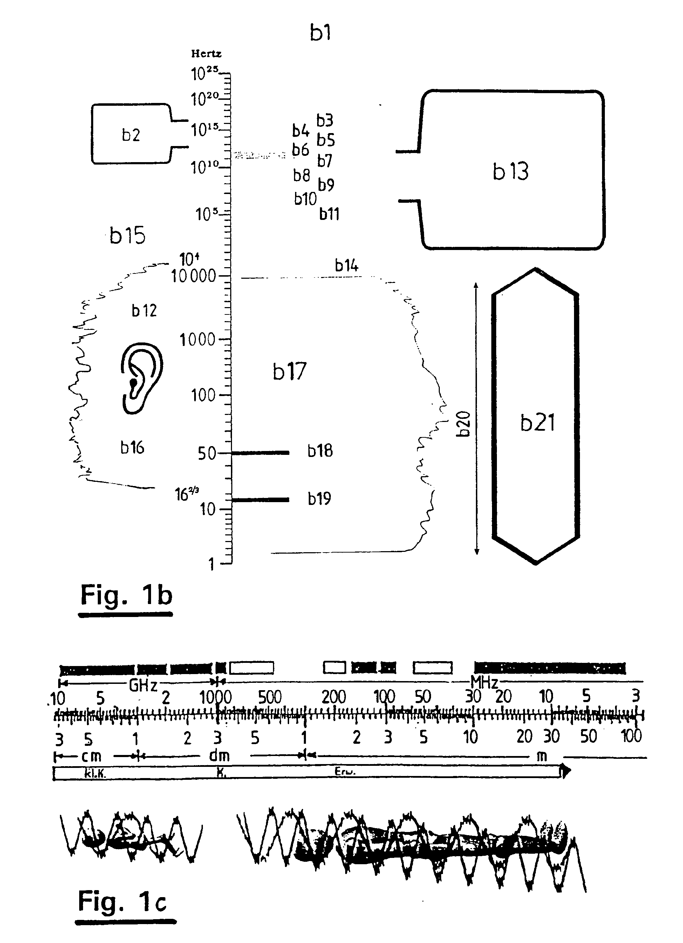

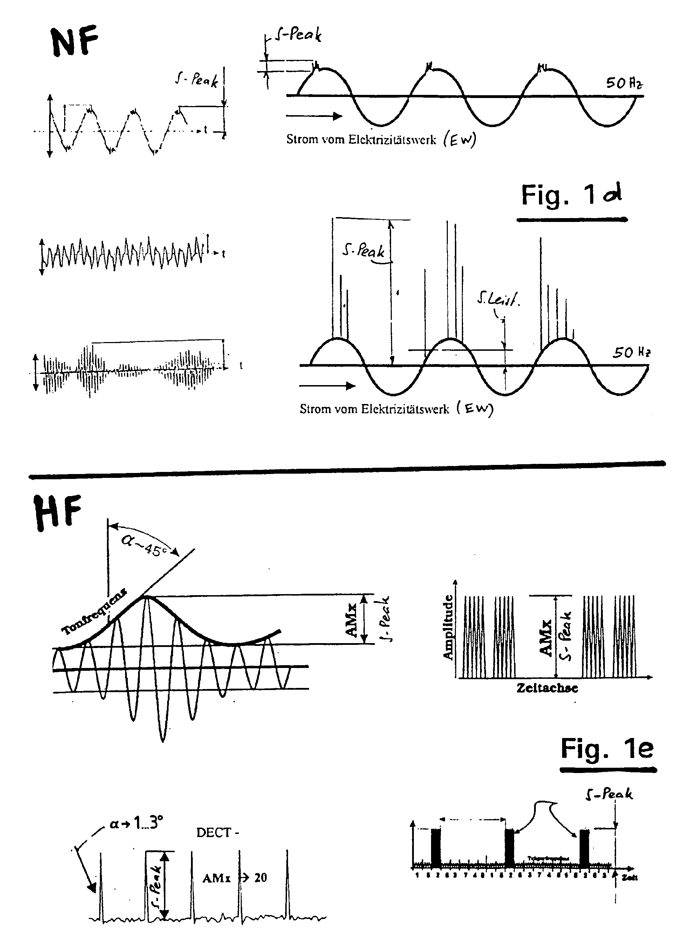

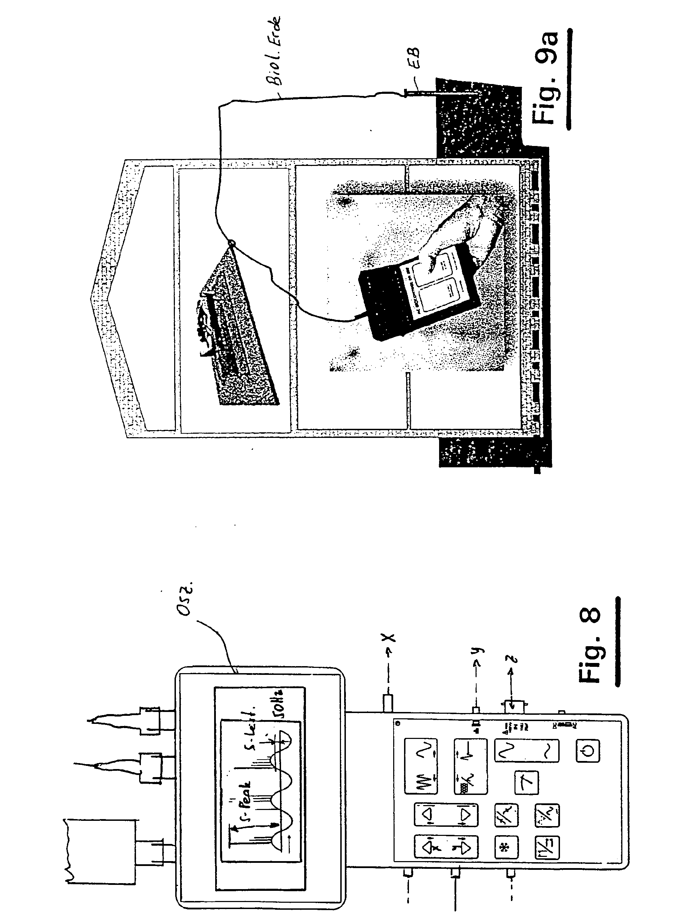

Die Figur 1 gibt einen Überblick über die neue Hausmesspraxis für das Ausmessen der Belastungen über den ganzen dargestellten Frequenzbereich. Im niederen Frequenzbereich ist im Zentrum die Ankopplungsspannung. Antenne ist bei der Ankopplungsspannung der ganze menschliche Körper. Bei niederen Frequenzen gelten allgemein die Gesetzmässigkeiten des Nahfeldes. Nahfeld (Nah.) = Abstand von der Quelle kleiner 1x Wellenlänge (λ) bzw. kleiner 3x Wellenlänge (λ). Kennzeichnend für das Nahfeldes ist die mögliche Trennung in den elektrischen sowie den magnetischen Teil. Dabei sind jedoch alle drei Möglichkeiten tägliche Realität, das verbündene elektromagnetische Feld, das elektrische Feld und das magnetische Feld. In der Figur 1a ist schematisch ein Voltmeter (Vol.) sowie ein Teslameter (Tes.) für die Magnetfeldmessung dargestellt. Für die Ankopplungsspannungs-messung links Mitte entspricht das Gerät den Figuren 8 bis 10. Hier hat das "Voltmeter" AC einen sehr hohen Eingangswiderstand, bevorzugt 1 ÷ 2 GΩ. Die bei-den Messaufbauten können in einem Gerät integriert oder als zwei unabhängig arbeitende Geräte ausgestaltet sein. Wichtig ist, dass alle Geräte batteriegespiesen sind, damit nicht vom Gerät selbst Störfelder erzeugt werden. Es ist ferner möglich die Störwirkung über einen entsprechenden Messaufnehmer sowie den Lautsprecher zu erfassen. Bevor-zugt werden das Ankopplungsspannungsmessgerät sowie der Hochfrequenzempfänger (rechts in der Figur 1 a) breitbandig mit überschneidenden Bereichen ausgebildet, so dass zwischen den beiden keine Lücke entstehen. Mit dem Hochfrequenzmessgerät (rechts, Mitte) wird die örtliche Hochfrequenzbelastung im Hinblick auf die elektrischen und magentischen Felder festgestellt. Damit ist es erstmals geglückt, den ganzen Bereich Elektrosmog mit kostengünstigen Handmessgeräten auszumessen, mit einer leicht verständlichen Spannungsmessung, so dass diese rasch lernbar und anwendbar ist. Ein ganz wichtiger Vorteil liegt darin, dass die vier kombinierten Methoden einander ergänzen und eine gegenseitige Kontrollfunktion haben. Es kann mit minimalem Zeitaufwand eine wirksame Sanierungsstrategie festgelegt und die Situation vor- und nachher überprüft werden. Beim HF-Messgerät bedeutet Res. A Resonanzantenne, Magn. A. Magnetfeldantenne, Ton der Lautsprecher für die ELF-Modulation, Digit. die digitale Anzeige der Feldstärke. Die Beschriftungen auf dem linken mittleren Bildteil der Figur 1a bedeuten Magn. die klassische Messung des Wechselmagnetfeldes über eine Spule (Spu.). Der Wert kann über ein Voltmeter (Vol) oder direkt über ein Teslameter mit der entsprechenden Anzeige in Tesla ermittelt werden. Direkt darunter ist die Erfassung des niederfrequenten elektrischen Feldes (Elekt). Sens bedeutet einen entsprechenden Messaufnehmer der die Person kontaktiert. Es handelt sich um die Messung der Körperspannung als Aufladung gegenüber biologischer Erde (Biol Erde). Strichpunktiert sind Messverbindungen zu einem Oszilloskop (Osz) dargestellt, das im unteren Bildteil angeordnet ist. Das Oszilloskop kann so ausgebildet sein, dass es sowohl für die niederwie hochfrequente Seite benutzbar ist. Es kann auch nur für die eine oder andere Funktion ausgelegt werden. Im Zentrum des Oszilloskopes ist die bildhafte Darstellung. Dabei sind alle klassischen Darstellungen des Standes der Technik möglich, jedoch nicht zwingend. Im Vordergrund ist die Darstellung von Oberschwingungen sowie den weiter oben beschriebenen Informationssignalen auf der niederfrequenten Seite sowie den sogenannten ELF-Signalen der hochfrequenten Seite. Mit X sowie Y sind zwei Anschlussmöglichkeiten an weitere Geräte z.B. zum Ausdrucken der Werte oder des Signalverlaufes und/oder zum Speichern der entsprechenden Werte, was mit Osz, Spu, Comp sowie Graph angedeutet ist.

Die Figur 1b gibt eine Übersicht über die verschiedenen Frequenzbereiche, wobei speziell im oberen Bildteil (eckiger Kasten) der Bereich von 10 Megahertz bis 10 Gigahertz hervorgehoben ist. Dieser Bereich ist der Frequenzbereich, in welchem Mensch (Tier und Pflanze) in Bezug auf elektromagnetische Wellen resonanzfähige Organe, bzw. Glieder besitzt. Darunter ist im Sechseck der sogenannte ELF-Bereich dargestellt. Es ist der wichtigste und empfindliche Bereich in dem die Bioelektronik aller Lebewesen arbeitet; und "effizient" gestört werden kann. Der ELF-Bereich entspricht etwa dem Hörbereich und dem Bereich der sogenannten Sferics-Basisstrahlung (0 - 15 KHz). Die Figur 1c ist eine Gegenüberstellung des angesprochenen Resonanzbereiches sowie der Körpergrösse eines Kleinkindes, eines Kindes sowie eines Erwachsenen. Dies bedeutet, dass Kleinkinder (auch Embryos) sehr viel stärker exponiert sind in dem höchsten Frequenzbereich.

Die Figur 1d zeigt links übereinander bekannte NF-Signale: klassische Störungen aus Haushaltsmaschinen (oben), Nähmaschine (mitte) sowie aus einem Laptop (unten). Rechts oben ist ein Steuersignal der Stromversorgung für das Schalten z.B. von elektrischen Haushaltwärmeverbrauchern. Darunter ist ein Störsignal, wie es z.B. in jüngster Zeit von der Datenübertragung über dem Netzstrom d.h. über der 50Hz-Netzschwingung feststellbar ist. Die Signalfolgen in der Figur 1d haben als Basis oder Träger eine niederfrequente Schwingung. Demgegenüber zeigt die Figur 1e niederfrequente Signalverläufe, welche von hoch- und höchstfrequenten Trägerfrequenzen als Immissionen messbar sind. Links oben im Bild ist die klassische Form der Amplitudenmodulation mit relativ flachem Flankenanstiegswinkel (α). Beispiel dafür ist ein Kurzwellensender. Rechts davon ist das typische Bild einer Radarimmission, links unten dasjenige eines sogenannten Dect-Telefones und rechts davon aus dem Mobilfunk. Die entsprechenden Signalverläufe können entweder akustisch oder bildlich dargestellt werden, da sie im sogenannten Hörbereich liegen.

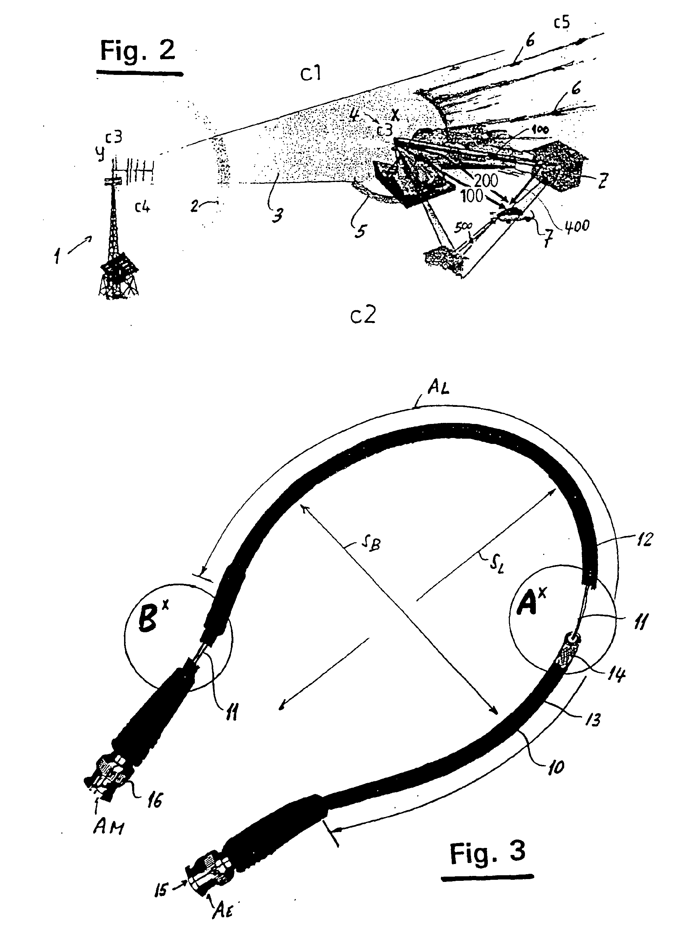

In der Figur 2 ist schematisch das Ineinanderspiel der Strahlenausbreitung von zwei Sendern eines UKW-Senders 1 (angenommen als Grosssender Y) mit einem ringförmigen Kreis 2 als den Bereich des Nahfeldes von dem Sender 1. Als zweiter Sender ist ein Sender X in der Annahme als Mobilfunkturm 4 mit Kreis 5 für die Begrenzung des Nahfeldes dargestellt. Die Sendesignale des Senders Y breiten sich im Nahfeld in Strahlrichtung aus. Ausgehend von dem Sender Y ist eine mächtige Sendekeule 3 dargestellt, welche den Sender X überstrahlt. Im Nahfeld des Senders X herrschen nur innerhalb eines Kreises mehr oder weniger kontrollierte Bedingungen. Sobald die Sendestrahlen des Senders X den kreisförmigen Ring 5 verlassen, beginnen unkontrollierte Verhältnisse. Die Mikrowellenstrahlung "hockt" nunmehr gleichsam auf den Seitenbändern der Sendekeule 3 auf und wird im Sinne eines Gratistransportes mitgezogen. Da es sich beim Sender X um sehr kurzwellige Strahlung handelt, ergibt sich noch vielmehr als bei Radiowellen eine Auffächerung der Strahlenbündel. Es entstehen Kleinkeulen und Kleinstkeulen 6, welche nunmehr durch die Seitenbänder der Sendekeule 3 mitgezogen werden. Die Sendesignale des Senders X sind z.B. für den Fahrer des Wagens 7 bestimmt. Mit der spezifischen Pulssendetechnik wird erreicht, dass die verschiedenen Sendepfade der Mobilfunksendestrahlen einander nicht stören. 100 bedeutet der direkteste Empfang, 200 eine Reflexion von nahem Berg, 300 von der weiter entfernt liegenden Hauswand und 400 von der näherliegenden Hauwand. Unweigerlich erhält nun aber das Haus Z neben dem Sendestrahl 3 des Senders Y, Kleinkeulen 6 und auch den Strahl 300 des Senders X und darüber hinaus eine beliebige Menge und in beliebiger Stärke Sendestrahlen von weiteren nicht dargestellten Sendern, stellvertretend durch den Strahl 500 dargestellt. Die Frage stellt sich sofort, was passiert aus der Menge von verschiedenen Strahlen mit unterschiedlichen Frequenzen in den Räumen des Hauses Z? Die Antwort dafür wurde weiter vorne gegeben. Es können sich ganz spezielle Feldverhältnisse, insbesondere die eingangs beschriebenen "Hot-spots" sowie Störballungen bilden. Mit welchen Hilfsmitteln diese detektiert und analysiert werden, wird mit den nachfolgenden Beispielen erläutert.

Die Figur 3 zeigt eine Schleifenantenne. Auf die allgemeinen Antennentheorien wird nicht im Detail eingegangen. An sich ist es bekannt, dass dutzende von Variationen möglich und einsetzbar sind. Bei der Figur 3 handelt es sich um die Bestform auf Grund der bisherigen Untersuchungen. Die Schleifenantenne hat drei charakteristische Grössen, die totale wirksame Länge AL des "Antennendrahtes" bzw. der Seele 11 des abgeschirmten Kabels 12, der Schleifenlänge SL sowie der Schleifenbreite SB. Im Falle eines Kreises sind SL und SB gleich gross. Mit Kreis Ax ist ein Ausschnitt in dem abgeschirmten Kabel zeichnerisch dargestellt. Mit schwarz ist als äusserste Haut ein Schrumpfschlauch 13 angebracht, der über eine isolierschicht oder direkt über einen Abschirmmantel 14 gezogen ist. Die Seele 11 ist die Empfangsantenne und erfasst durch die Abschirmung einerseits sowie die Schleifenform betont oder ausschliesslich die magnetische Feldkomponente. Über dem rechten Anschluss AE wird die Seele als Messeingang an die Elektronik geführt, wohingegen die Abschirmung 14 mit der Gerätemasse verbunden wird. Durch Aufschrauben der Bajonettverbindungen 15 und 16 an den gewünschten Antennenanschluss wird die Antenne an ein Messgerät angeschlossen. Über dem linken Anschluss AM wird die Seele 11 direkt mit der Gerätemasse verbunden. Daraus ergibt sich eine wirksame Antennenlänge AL wie eingezeichnet. Wie erwähnt handelt es sich bei der gezeigten Lösung nur um ein Beispiel. Es ist absehbar, dass mit grösseren Versuchsreihen insbesondere mit sehr viel mehr Praxiserfahrung bzw. entsprechender Rückmeldung der konstruktive Aufbau noch stark optimierbar ist, auch im Rahmen der bekannten Magnetfeldmessantennen auch mit geschlossenem Ring und nur einer Bajonettverbindung. Nach allen bisherigen Erfahrungen weisen die Störfeldballungen jede Art von Strahlen- und Keulen bis zu Ballformen auf. Grossmehrheitlich ist den örtlichen Ballungen gemeinsam, dass der magnetische Feldanteil dominiert. Was bis heute vollkommen übersehen wurde, ist die Tatsache, dass sehr häufig die magnetische Amplitudenmodulation dominiert. Die "Musik" aus dem Lautsprecher lässt sofort die Art der Störsignaltechnik zu erkennen. Bei der neuen Lösung können auch die klassischen, geschlossenen Magnetfeldringantennen anstelle der teilweise offenen Schleifen- bzw. Ringformen verwendet werden. Die offene Form hat vor allem auch praktische Vorteile, z.B. der leichteren Unterbringung in eine Messtasche. Der geschlossene Ring ist dagegen empfindlicher in Bezug auf eine Deformierung.

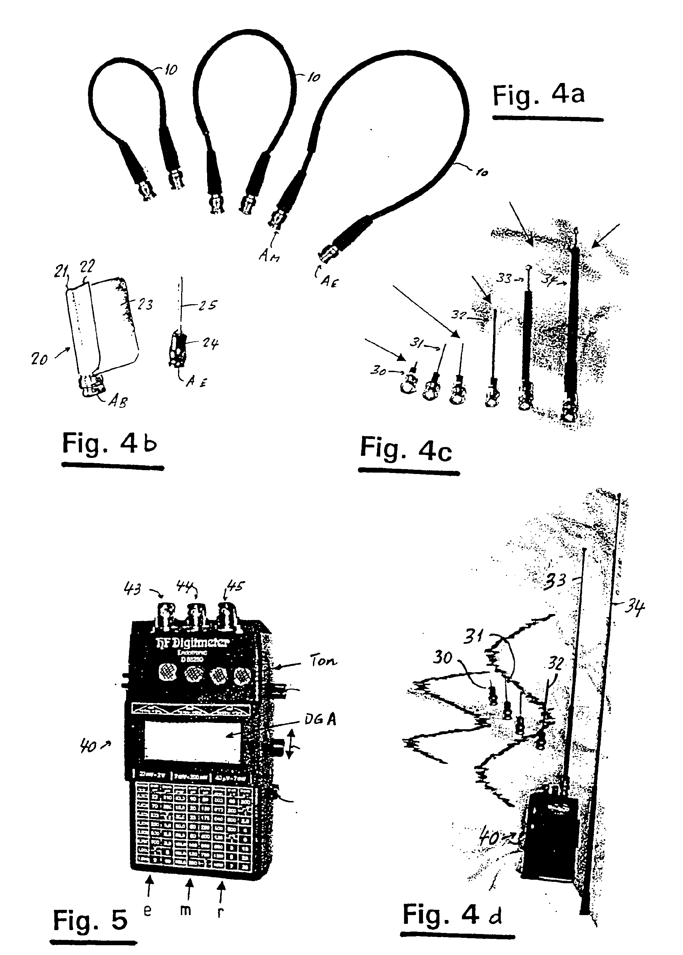

Die Figuren 4a, 4b, 4c und 4d zeigen ein komplettes Antennenset für eine Hausmessung, wobei die Figur 4a drei verschiedene Grössen von Schleifenantennen darstellt. Die Figur 4b zeigt eine sehr einfache Peilantenne 20, für die Ermittlung der Strahlenrichtung. Die Peilantenne 20 besteht aus zwei Teilen, einem Peiltrichter 21, mit zwei kleinen etwa 30 bis 40° zueinander geöffneten Reflektorplatten 21 und 22 sowie einer Bajonetverbindung AB, welche eine blosse Haltefunktion für den Peiltrichter hat. Eine kurze etwa 4,5 cm lange Drahtantenne 25 mit Bajonettverbindung AE stellt das eigentliche Aufnahmeteil 24 für die Peilantenne 20 dar. Der Peiltrichter 21 hat eine doppelte Funktion. Er soll - mit Ausnahme der offenen Seite - die Drahtantenne 25 vor unerwünschten Einstrahlungen schützen. Dagegen wirkt der Trichter durch die beiden Reflektorwände wie ein Fang, so dass mit einem Antennengewinn in Richtung der Mittenachse des Fangtrichters die grösstmöglichen Werte gemessen werden, wenn die Richtung genau auf eine Quelle gerichtet ist. Dabei wird eine keulenartige Strahlwirkung vorausgesetzt. Die weiter oben beschriebenen Störballungen, ohne direkt erkennbare Einstrahlrichtung lassen sich mit der Peilantenne nicht näher bestimmen, ausser in Bezug auf die ELF-Modulationen. Wichtig ist in jedem Fall, dass neben der Intensität auch die "Qualität" der Störwirkung ermittelt wird. Die Figur 4c zeigt ein ganzes Set von an sich bekannten Resonanzantennen, wie sie von den Anmeldern seit vielen Jahren zu HF-Messgeräten hergestellt werden. Die kleinste Antenne 30 mit 1 cm Länge als Stechantenne oder für Richtstrahl, die 4,5 cm lange Mikrowellenantenne 31 z.B. auch für Mobilfunk D + E, die 9 cm Antenne für Mobilfunk sowie Bildschirmsteuersignale sowie die ausziehbaren Antenne 33 und 34 für Radiofrequenzen.

Die Figur 4d und 5 zeigen einen kombinierten Hochfrequenzempfänger 30, mit einer Digitalanzeige DGA für die Messwerte. Oben am Gerät sind verschiedene Anschlüsse, wobei eine Antenne 33 (Figur 4d) an dem rechten Anschluss 2 aufgesetzt ist. Im unteren Teil erkennt man einen Lautsprecher (Ton), über den das über die Antenne empfangene ELF-Signal akustisch wiedergegeben wird, z.B. die typischen Signaltöne eines Natelempfängers. Um die verschiedenen Wellenlänger zumindest bereichsweise gesondert messen zu können, wird bevorzugt ein kleines Sortiment an verschieden langen Antennen 30 - 34 auf das Gerät aufsetzbar bereitgestellt. Wie mit den Wellenformen Figur 4d angedeutet ist, wird, je nach dem, welche Frequenzen dominant vorhanden sind, die passende Antennenlänge aufgesetzt, dies nach den bekannten Antennengesetzen mit λ*,* λ/2, λ/4. Über die Länge der Antenne lässt sich der Frequenzbereich und über den Lautsprecher die Art der Signalübertragung (analog/ digital gepulst usw.) feststellen. Man erhält damit auf sehr einfache Weise die Antennenspannung, den Frequenzbereich sowie die Signaltechnik gleichzeitig und kann gemäss Richtwerttabelle (Figur 1) die örtliche Belastung feststellen und gegebenenfalls die Situation zielgerichtet sanieren. Die Figur 5 zeigt ein HF-Messgerät 40, welches über eine Digitalanzeige, die örtliche HF-Feldbelastung bevorzugt in der Form der Antennenspannung anzeigt, und über Lautsprecher 42 die Elf-Modulationen akustisch wiedergibt. Je lauter die akustische Anzeige desto intensiver die Elf-modulierten Signale bzw. die Peaks. Ein besonderes Merkmal des HF-Messgerätes 40 sind drei Antennenanschlussmöglichkeiten, die folgende Bereiche aufweisen: Linker Anschluss 43: 22 mV - 2 Volt Antenneneingang , 10 kHz - 4 GHz, als Eingang mit geringster Empfindlichkeit; mittlerer Anschluss 44: 10 mV - 200 mV Antenneneingang, 10 kHz - 4 GHz; rechter Anschluss 45: 40 *µ*V - 7 mV, Antenneneingang, 10 MHz - 4 GHz, Eingang mit höchster Empfindlichkeit. Wichtig ist nun, dass jede Antenne an jedem Anschluss anschliessbar ist, so dass nach Betätigung des entsprechenden Bereichsschalters alle Funktionen des Gerätes über jede Antenne mit dem selben Messgerät nutzbar ist. Gleichgültig ob die Peilantenne, die Schleifenantenne oder eine Resonanzantenne aufgesetzt wird, in jedem Fall erhält man sowohl den digitalen Messwert als Antennenspannung (oder umgerechnet als magnetische oder elektrische Feldstärke in (Volt/m) und über die Akustik die Modulationsart und Intensität. Jede Antenne gibt die ganze Information, die auf Grund des Antennenbaues möglich ist. Die Antennenform kann auch 3- oder vieleckig gestaltet sein.

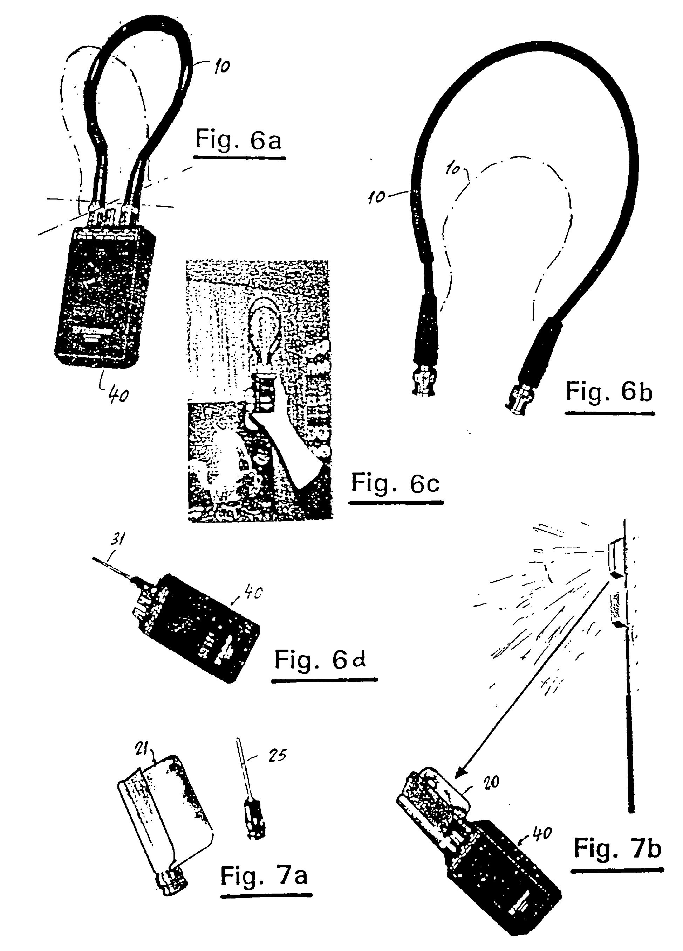

Die Figuren 6a, 6b und 6c zeigen ein Beispiel für den Einsatz der Schleifenantennen. In der Figur 6a ist strichpunktiert angedeutet, dass zwei Antennen auch kreuzweise einsetzbar sind, vorausgesetzt, die Anschlüsse sind entsprechend ausgebildet. Die Figur 6c zeigt drei verschiedene Schleifenantennengrössen in der Grössenordnung von 20, 40 und 40 cm Drahtlänge. Bei der grössten Schleifenantenne ist eine kleine Antenne strichpunktiert angedeutet, die gleichzeitig oder mit Umschalter wechselweise nutzbar sind. Die Figur 6d zeigt das selbe HF-Messgerät mit aufgesetzter 4,5 cm langer Resonanzantenne, für eine exakte Analyse spezifischer Mikrowellenstrahlung wie z.B. Haustelefon, Mobilfunk oder Mikrowellenofen. Es lassen sich beliebige Antennenkonfigurationen, gegebenenfalls mit Umschaltern einsetzen, seien es mehrere ineinander oder kreuzweise.

Die Figur 7a und 7b zeigen schematisch die Funktion der Anpeilung einer Quelle mit der Peilantenne für das rasche Anbringen einer Abschirmung bzw. die Kontrolle der Abschirmung in einem Raum.

Die Figur 8 zeigt ein batteriebetriebenes Oszilloskops Osz. Am Oszilloskop sind im Oberteil Anschlussmöglichkeiten für den direkten Anschluss von Messaufnehmern oder z.B. direkt für Antennen (gemäss Figuren 4a bis 4d). Neben den klassischen Funktionen eines Oszilloskopes ist für die neue Lösung der Peak-Wert sowohl bei einem niederfrequenten Signal wie auch bei der ELF-Modulation von Interesse. Die Leistungsangabe SLeist, hat in Bezug auf die Peak-Werte nur relativen Wert. Interessant ist aber auch die zeitliche Folge, die Häufigkeit und der Stunden oder Tagesverlauf der Peak-Werte. Für die Beurteilung der Peak-Werte ist der tatsächliche Spitzenwert massgebend und nicht etwa ein 6-Minuten-Mittelwert. Die Art, Höhe und Häufigkeit der Peak-Werte sind von besonderer Relevanz für die biologische Wirkung. Es liegt auf der Hand, dass für die Auswertung alle erfassten Messwerte berücksichtigt gegebenenfalls gespeichert ausgedrückt und ausgewertet werden sollen.

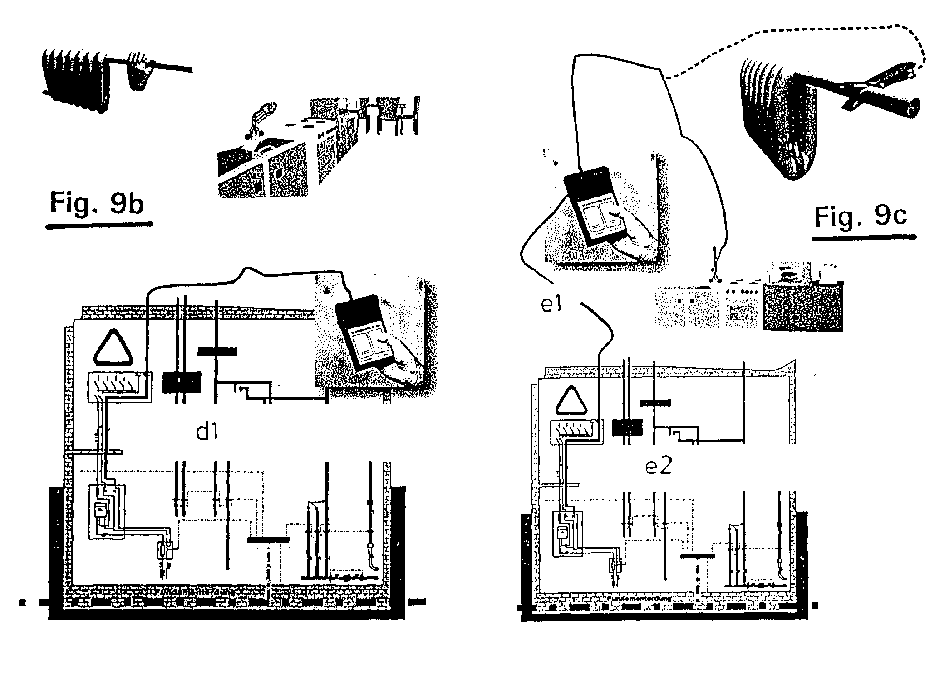

Die Figur 9a zeigt bildhaft das Wunschziel der Störfeldfreiheit, wobei sowohl für die Messung wie für einen Schlafplatz die Wirkung der biologischen Erdung genutzt wird. Diese wird über einen Erdbohrer EB ausserhalb des Gebäudes im Erdboden über einem Erdbohrer geholt. An der Schlafstelle sollten nachts keine Störfelder vorhanden sein und möglichst biologische Erdungsverhältnisse bestehen. Gleicherweise soll für eine exakte Messung der Ankopplungsspannung in Bezug auf eine biologische Erde als Potential Null gemessen werden. Ausserhalb dem Gebäude ist eine biologische Erdung E_{B} mittels eines Pfahles als Oberflächenerdung gezeichnet. Die Figur 9b zeigt gemäss der neuen Strategie die einfachste Kontrolle der Erdungspotentiale. Für die Messung wird die Geräteerde an den Schutzleiter der elektrischen Installation angeschlossen. Das Gerät wird mit einer Hand betätigt bzw. der Sensor gleichzeitig aktiviert. Mit der anderen Hand kontaktiert die selbe Person nacheinander z.B. eine Brauchwasserleitung und eine blanke Stelle eines Heizungsradiators. Besteht kein Potentialunterschied, so kann die anschliessende Ausmessung gegenüber einem der drei Anlageteile durchgeführt werden. Die Figur 9c zeigt in Analogie zu der Figur 9b wie rein gerätetechnisch jedoch ohne Zwischenschaltung des Menschen über die entsprechende Aus- bzw. Eingänge am Messgerät die Potentiale verglichen bzw. gemessen werden können.

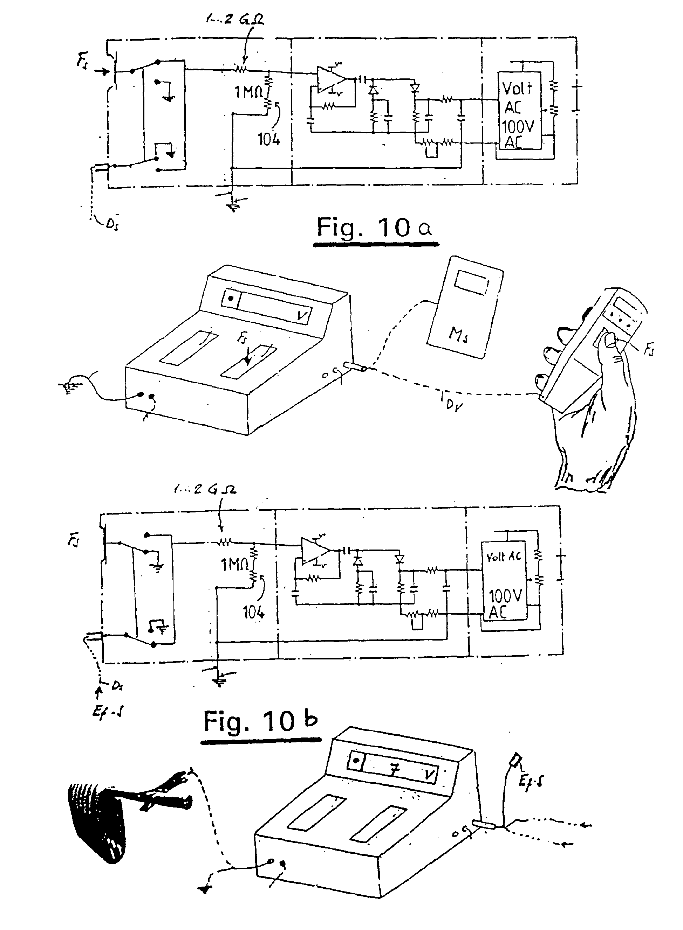

Die Figuren 10a und 10b zeigen ein kombiniertes Gerät, über das sowohl die Ankopplungsspannung am Körper des Menschen wie auch die unterschiedlichen Potentiale bzw. Erdpotentiale zwischen verschiedenen Bauteilen gemessen werden können. Die Figur 10a entspricht der Lösung der WO96/35371, weshalb auf diese Druckschrift Bezug genommen wird. Neben der Betätigung über einen Fingersensor Fs kann zusätzlich oder alternativ über einen geschützten Sensor, der die empfindlichen Teile der Elektronik integriert hat, die Ankopplungsspannung gemessen werden. Über eine Drahtverbindung Dv können die Messwerte am Gerätedisplay (V) digital angezeigt werden. Da das Messgerät funktio-nell einem Voltmeter entspricht, kann ferner über ein Magnetfeldaufnehmer (Ms) das örtliche Magnetfeld angezeigt werden. Ds bedeutet Drahtsonde. Dv Drahtverbindung. In der Figur 10b ist die Eingangsbeschaltung (links ein Schema in umgeschalteter Stellung gegenüber der Figur 10a dargestellt. Die entsprechende Eingänge sind entweder aktiviert oder mit Erde verbunden. In der Figur 10a ist zusätzlich die Möglichkeit des Einsatzes einer elektrischen Feldsonde Efs dargestellt. Diese Lösung dient in erster Linie dem Aufspüren von elektrischen Störfeldquellen bzw. dem Aufsuchen von verborgenen Leitungen über das elektrische Feld.

### Figur 1c:

- kl.K. =: Kleinkind
- K. =: Kind
- Erw. =: Erwachsener

### Figur 2:

- c1 =: Mächtige Sendekeulen, z.B. von UKW-Sendern bilden einen Gratistransport auch für Sendestrahlen wie Natelsender und dergleichen
- c2 =: Die lokalen Mikrowellenstrahlen können nicht nur über 1000 Reflexionen sondern auch über Grosssender auf sogenannten Seitenbändern mitgetragen und örtlich als zusätzliche massive Belastung auftreten
- c3 =: Sender
- c4 =: Nahfeld
- c5 =: Fernfeld

### Figur 9b:

- d1 =: Sind alle 3 gemessenen Potential identisch, so kann die Ausmessung von Arbeits - und Schlafstelle über den elektrischen Schutzleiter vorgenommen werden

### Figur 9c:

- e1 =: Geräteanschluss an den elektrischen Schutzleiter
- e2 =: Sind alle 3 gemessenen Potential identisch, so kann die Ausmessung von Arbeits - und Schlafstelle über den elektrischen Schutzleiter vorgenommen werden

### Textbeschrieb zu Figuren:

### Figur 1a:

- a1 =: Richtwerte für Langzeitbelastungen (bei Belastung aus verschiedenen Frequenzen)
- a2 =: ungepulst, analog
- a3 =: gepulst, digital
- a4 =: Höchst Elektrosensible

### Figur 1b:

- b1 =: Elektromagnetischer Bereich
- b2 =: Lichtbereich
- b3 =: Ultraviolett
- b4 =: sichtbares Licht
- b5 =: Infrarot
- b6 =: Mikrowelle
- b7 =: Dezimeterwelle
- b8 =: UKW
- b9 =: Kurzwelle
- b10 =: Mittelwelle
- b11 =: Langwelle
- b12 =: Hörbereich
- b13 =: Frequenzbereich, in welchem Menschen (Tier und Pflanze) in Bezug auf elektromagnetische Wellen resonanzfähige Organe, bzw. Glieder besitzt: 10 Megahertz bis 10 Gigahertz
- b14 =: Wetterstrahlung
- b15 =: Hörbereich
- b16 =: Tonfrequenzen
- b17 =: Sferics-Basisstrahlung wenig über 0 - max. 15 kHz
- b18 =: Netzfrequenz
- b19 =: Bahnfrequenz
- b20 =: ELF (extremly low frequency)
- b21 =: Wichtigster und empfindlicher Bereich in dem die Bioelektronik aller Lebewesen arbeitet, und "effizient" gestört werden kann.

## Patentansprüche

1. Verfahren zur Erfassung von hochfrequenten elektromagnetischen Störwechselfeldern über eine Antennenspannung mittels eines batteriegespeisten Messgerätes mit einer Eingangsempfindlichkeit bis in den Mikrovoltbereich, mit einer Digital- und/oder Bildanzeige und mit einem Antennenanschluss, mit folgenden Schritten:
a) Erfassung eines hochfrequenten Träger-Störwechselfeldes
b) Erfassung von Peakwerten des Störwechselfeldes
c) Erfassung einer niederfrequenten Modulation des Störwechselfeldes im Bereich von 0 bis etwa 15 kHz.

2. Verfahren nach Anspruch 1,
**dadurch gekennzeichnet,**
**dass** zusätzlich ein niederfrequentes Störwechselfeld hochohmig mittels Potentialdifferenzen bis herab in den Millivoltbereich gemessen wird und zusätzlich das Magnetfeld und/oder Peakwerte des niederfrequenten Störwechselfeldes erfasst werden.

3. Verfahren nach Anspruch 2,
**dadurch gekennzeichnet,**
**dass** im niederfrequenten Bereich Potentialdifferenzen zwischen störfeldbelasteten Massen oder einzelnen störfeldbelasteten Massen und Erdleitern über einen Eingangswiderstand von wenigstens 100 Mega Ω erfasst werden.

4. Verfahren nach Anspruch 1,
**dadurch gekennzeichnet,**
**dass** im hochfrequenten Bereich, insbesondere bei den cm- und dm- Trägerwellen, die Antennenspannung über einen Hochfrequenzempfänger breitbandig erfasst wird und als wenigstens einen zusätzlichen Störparameter die Art der Modulationstechnik und/oder der magnetische Feldanteill ermittelt wird.

5. Verfahren nach Anspruch 1 oder 4,
**dadurch gekennzeichnet,**
**dass** Orte unterschiedlicher Störfeldstärken infolge inhomogener Felder, insbesondere magnetische Feldmaxima ermittelt und das örtlich störwirksame Feld gleichzeitig in Bezug auf seine Trägerfrequenz und auf die niederfrequenten Anteile detektiert werden.

6. Verfahren nach einem der Ansprüche 1 bis 5,
**dadurch gekennzeichnet,**
**dass** örtliche Störfelder angezeigt und/oder hörbar gemacht und/oder zeitlich gespeichert werden und die gespeicherten Feldstärken in Bezug auf die Zeit registrierbar sind.

7. Spannungsmesseinrichtung mit Batteriespeisung für die Erfassung von hochfrequenten elektromagnetischen Störwechselfeldern über eine Antennenspannung mit einer Digital- und/oder Bildanzeige und mit einem Antennenanschluss,
**dadurch gekennzeichnet,**
**dass** die Messeinrichtung eine Eingangsempfindlichkeit bis in den Mikrovoltbereich aufweist und geeignet ist für die Erfassung der Peakwerte des Störwechselfeldes, wobei mit der Messeinrichtung sowohl
a) ein hochfrequentes Träger-Störwechselfeld als auch
b) eine niederfrequente Modulation des Störwechselfeldes im Bereich von 0 bis etwa 15 kHz erfassbar ist.

8. Spannungsmesseinrichtung nach Anspruch 7,
**dadurch gekennzeichnet,**
**dass** eine Schleifenantenne anschliessbar ist für den Megahertz- bis 10 Gigahertzbereich, welche als nicht abgestimmte Magnetfeldantenne ausgebildet ist.

9. Spannungsmesseinrichtung nach Anspruch 8,
**dadurch gekennzeichnet,**
**dass** die beiden Enden der Schleifenantenne zur Stabilisierung der Schleifenform an zwei versetzt angeordneten Antennenanschlüssen anschliessbar sind.

10. Handmesssystem, bestehend aus einer Spannungsmesseinrichtung nach Anspruch 7 bis 9 und einer Schleifenantenne,
**dadurch gekennzeichnet,**
**dass** die Schleifenantenne als abgeschirmtes Leiterstück ausgebildet ist, mit einer Abschirmung in Bezug auf ein elektrisches Feld wobei die Schleifenantenne etwa in Handgrösse ausgebildet ist, und eine runde oder elliptische Form hat, und/oder dass zwei oder mehrere Schleifenantennen mit einer wirksamen Antennendrahtlänge in dem Bereich von 10 bis 60 cm einsetzbar sind.

11. Handmesssystem bestehend aus einer Spannungsmesseinrichtung nach Anspruch 7 bis 9 sowie einer Antenne, wobei die Antenne als geschlossene Magnetfeldringantenne oder als offene Schleifenantenne oder als Resonanzantenne oder als Peilantenne ausgebildet ist.

12. Spannungsmesseinrichtung nach Anspruch 7,
**dadurch gekennzeichnet,**
**dass** ein Oszilloskop anschliessbar ist, zur Anzeige der erfassten Modulation oder dass die Spannungsmesseinrichtung zusätzlich als Oszilloskop ausgebildet ist.

13. Verwendung der messeinrichtung nach einem der Ansprüche 7 bis 9, oder des Handmesssystems nach Anspruch 10, für die Messung und Überwachung der Immission in Bezug auf die Zeit sowie die drahtlose Sendetechnik wie Mobilfunk, drahtlose Haustelefone, TV-Sendestrahlung und den übrigen Sendesystemen im Megahertzbereich bis zu einem Bereich 10 Gigahertz, insbesondere mit akustischer und/oder bildhafter Anzeige der Modulation und im besonderen der Peak-Werte.

## Claims

1. Method for detecting high-frequency electromagnetic interference alternating fields via an antenna voltage using a battery-powered measuring device having an input sensitivity of up to the microvolt range, comprising a digital and/or image display and an antenna terminal, involving the following steps:
a) detecting a high-frequency carner-interference alternating field,
b) detecting peak values in the interference alternating field,
c) detecting a low-frequency modulation in the interference alternating field in the range of from 0 to approximately 15 kHz.

2. Method according to claim 1, **characterised in that** a low-frequency interference alternating field is also measured in a high-resistance by potential differences down into the millivolt range and the magnetic field and/or peak values of the low-frequency interference alternating field are also detected.

3. Method according to claim 2, **characterised in that** in the low-frequency range, potential differences are detected between interference field-loaded earths or individual interference field-loaded earths and earth leads via an input resistor of at least 100 mega Ω.

4. Method according to claim 1, **characterised in that** in the high-frequency range, in particular for the cm- and dm-carrier waves, the antenna voltage is detected in a broadband manner via a high-frequency receiver and the type of modulation method and/or the magnetic field proportion is determined as at least an additional interference parameter.

5. Method according to either claim 1 or claim 4, **characterised in that** locations of different interference field intensities, in particular magnetic field maximum values, caused by inhomgeneous fields, are determined and the local disturbing field is detected simultaneously in respect of the carrier frequency thereof and the low-frequency proportions.

6. Method according to any one of claims 1 to 5, **characterised in that** local interference fields are displayed and/or are made audible and/or are stored with respect to time and the stored field intensities may be registered with respect to time.

7. Voltage measuring device with battery power supply for detecting high-frequency electromagnetic interference alternating fields via an antenna voltage comprising a digital and/or image display and comprising an antenna terminal, **characterised in that** the measuring device has an input sensitivity into the microvolt range and is suitable for detecting the peak values of the interference alternating field, said measuring device allowing the detection of both
a) a high-frequency carrier interference alternating field and
b) a low-frequency modulation of the interference alternating field in the range of from 0 to approximately 15 kHz.

8. Voltage measuring device according to claim 7, **characterised in that** a frame antenna may be connected for the megahertz to 10 gigahertz range and is configured as a non-tuned magnetic field antenna.

9. Voltage measuring device according to claim 8, **characterised in that** for stabilising the frame antenna, the two ends of the frame antenna may be connected to two staggered antenna terminals.

10. Manual measuring system, consisting of a voltage measuring device according to any one of claims 7 to 9 and of a frame antenna, **characterised in that** the frame antenna is configured as a shielded conductor piece, having a shielding in respect of an electric field, the frame antenna approximately the size of a hand, and the frame antenna has a round or elliptic shape, and/or **in that** two or more frame antennae may be used which have an effective wire length ranging from 10 to 60 cm.

11. Manual measuring system, consisting of a voltage measuring device according to any one of claims 7 to 9 and of an antenna, wherein the antenna is configured as a closed magnetic field frame antenna or as an open-loop antenna or as a resonant antenna or as an arrow antenna.

12. Voltage measuring device according to claim 7, **characterised in that** an oscilloscope may be connected to display the detected modulation or **in that** the voltage measuring device is also configured as an oscilloscope.

13. Use of the voltage measuring device according to any one of claims 7 to 9 or of the manual measuring system according to claim 10, for measuring and monitoring the emission with respect to time and the wireless transmitting method such as mobile radio communications, cordless house telephones, TV transmission radiation and other transmission systems in the megahertz range up to a range of 10 gigahertz, in particular with acoustic and/or visual display of the modulation and in particular of the peak values.

## Revendications

1. Procédé pour détecter des champs alternatifs perturbateurs électromagnétiques à haute fréquence par l'intermédiaire d'une tension d'antenne et au moyen d'un appareil de mesure alimenté par batteries avec une sensibilité d'entrée allant jusque dans la plage des microvolts qui comprend un affichage digital et/ou par images et un branchement d'antenne, les étapes du procédé étant les suivantes :
a) Détection d'un champ alternatif perturbateur porteur à haute fréquence
b) Détection de valeurs crêtes du champ alternatif perturbateur
c) Détection d'une modulation à basse fréquence du champ alternatif perturbateur dans la plage de 0 à environ 15 kHz.

2. Procédé selon la revendication 1,
**caractérisé en ce que**
de surcroît un champ alternatif perturbateur à basse fréquence est mesuré avec une valeur ohmique élevée au moyen de différences de potentiel descendant jusque dans la plage des millivolts et que de surcroît le champ magnétique et/ou des valeurs crêtes du champ alternatif perturbateur à basse fréquence sont détectés.

3. Procédé selon la revendication 2,
**caractérisé en ce que**
dans la zone à basse fréquence, des différences de potentiel entre des masses chargées par le champ perturbateur ou entre des masses individuelles chargées par le champ perturbateur et des conducteurs de terre sont détectées par l'intermédiaire d'une résistance d'entrée de moins de 100 mégaohms.

4. Procédé selon la revendication 1,
**caractérisé en ce que**
dans la zone à haute fréquence, en particulier dans le cas des ondes porteuses centimétriques et décimétriques, la tension d'antenne est détectée dans une large bande par l'intermédiaire d'un récepteur à haute fréquence et **en ce que** le genre de technique de modulation et/ou la part magnétique du champ sont déterminés en tant qu'au moins un paramètre perturbateur supplémentaire.

5. Procédé selon la revendication 1 ou 4,
**caractérisé en ce que**
des endroits avec des intensités différentes des champs perturbateurs, en particulier des maxima magnétiques des champs, sont déterminés du fait de champs non homogènes et **en ce que** le champ localement actif en perturbation est détecté en même temps en fonction de sa fréquence porteuse et des parts à basse fréquence.

6. Procédé selon l'une quelconque des revendications 1 à 5,
**caractérisé en ce que**
des champs perturbateurs locaux sont affichés et/ou sont rendus audibles et/ou sont mémorisés chronologiquement et **en ce que** les intensités de champs mémorisées peuvent être enregistrées en fonction du temps.

7. Dispositif de mesure de tension alimenté par batterie pour la détection de champs alternatifs perturbateurs électromagnétiques à haute fréquence par l'intermédiaire d'une tension d'antenne qui comprend un affichage digital et/ou par images et un branchement d'antenne,
**caractérisé en ce que**
le dispositif de mesure a une sensibilité d'entrée allant jusque dans la plage des microvolts et est adapté pour la détection des valeurs crêtes du champ alternatif perturbateur, le dispositif de mesure permettant de détecter aussi bien
a) un champ alternatif perturbateur porteur à haute fréquence que
b) une modulation à basse fréquence du champ alternatif perturbateur dans la plage de 0 jusqu'à environ 15 kHz.

8. Dispositif de mesure de tension selon la revendication 7,
**caractérisé en ce qu'**il est possible de raccorder une antenne à cadre pour la plage des mégahertz jusqu'à 10 gigahertz, laquelle est configurée comme antenne de champ magnétique non accordée.

9. Dispositif de mesure de tension selon la revendication 8,
**caractérisé en ce que**,
pour la stabilisation de la forme du cadre, les deux extrémités de l'antenne à cadre peuvent être raccordées à deux connecteurs d'antenne disposés avec un décalage.

10. Système de mesure manuel se composant d'un dispositif de mesure de tension selon les revendications 7 à 9 et d'une antenne à cadre,
**caractérisé en ce que**
l'antenne à cadre est configurée pièce conductrice blindée avec un blindage contre un champ électrique, l'antenne à cadre étant configurée à peu près de la taille d'une main et ayant une forme ronde ou elliptique, et/ou **en ce qu'**il est possible de mettre en place deux ou plusieurs antennes à cadre avec une longueur efficace du fil d'antenne située dans la plage de 10 jusqu'à 60 cm.

11. Système de mesure manuel se composant d'un dispositif de mesure de tension selon les revendications 7 à 9 ainsi que d'une antenne, l'antenne étant configurée comme antenne annulaire de champ magnétique fermée ou comme antenne à cadre ouverte ou comme antenne à résonance ou comme antenne radiogoniométrique.

12. Dispositif de mesure de tension selon la revendication 7,
**caractérisé en ce qu'**il est possible de raccorder un oscilloscope pour afficher la modulation détectée ou en ce que le dispositif de mesure de tension est de surcroît configuré comme oscilloscope.

13. Utilisation du dispositif de mesure de tension selon l'une quelconque des revendications 7 à 9 ou du système de mesure manuel selon la revendication 10 pour la mesure et la surveillance de l'immission par rapport au temps ainsi que pour les techniques d'émission sans fil telles que la radiophonie mobile, les téléphones domestiques sans fil, la diffusion des émissions de télévision et les autres systèmes d'émission travaillant dans la plage des mégahertz jusqu'à la plage des 10 gigahertz, en particulier avec un affichage acoustique et/ou par l'image de la modulation et notamment des valeurs crêtes.
